# EUROPEAN PATENT SPECIFICATION

(11) **EP 2 590 576 B1**
(45) Date of publication and mention of the grant of the patent: **14.10.2015**
(21) Application number: 11803024.6
(22) Date of filing: 07.07.2011
(51) Int. Cl.: A61B 17/06

(54) **COMPRESSION BONE SCREW**
KOMPRESSIONSKNOCHENSCHRAUBE
VIS OSSEUSE À COMPRESSION

(30) Priority: 07.03.2011 AU 2011900800; 07.07.2010 AU 2010903020
(43) Date of publication of application: 15.05.2013
(73) Proprietor: Yalizis, Matthew Adam, Sans Souci, NSW 2219 (AU)
(72) Inventor: Yalizis, Matthew Adam, Sans Souci, NSW 2219 (AU)
(74) Representative: Georgiou, Sarah Caroline
(86) International application number: PCT/AU2011/000865
(87) International publication number: WO 2012/003548

(56) References cited:
- EP-A1- 1 046 376
- WO-A1-97/22301
- WO-A1-2006/124987
- WO-A2-2004/069031
- WO-A2-2004/069031
- FR-A1- 2 699 065
- GB-A- 2 323 533
- US-A- 4 456 005
- US-A- 4 858 601
- US-A1- 2006 142 770
- US-A1- 2010 211 115

## Description

### Technical Field

The present invention relates to the field of bone fixation in orthopaedic surgery and in particular relates to a compression bone screw.

### Background of the Invention

Compression bone screws are used in orthopaedic surgery for bone fixation, and in particular to fix two fragments of a fractured bone and for fixation of osteotomies and arthrodeses. Healing of fractured bones is greatly improved with the application of compression between the bone fragments.

Various forms of compression bone screw have previously been proposed seeking to provide such compression between bone fragments. One form of known compression bone screw, known as the Herbert screw, utilises external leading and trailing threads on the screw for embedment within distal and proximal bone fragments. The leading and trailing external threads are like-handed, but are of unequal pitch, with a greater pitch on the leading thread. This advances the screw through the distal bone fragment at a greater rate than through the proximal bone fragment, thereby drawing the distal bone fragment towards the proximal bone fragment, providing a degree of compression between the bone fragments. The amount of compression provided, however, cannot be readily controlled and is entirely dependent upon the thread pitch differentiation and the depth to which the screw must be embedded to provide adequate purchase on the distal and proximal bone fragments.

Another previously proposed form of compression bone screw utilises separate externally threaded leading and trailing elements for embedment within the distal and proximal bone fragments. The leading and trailing elements are rotatable independently of each other and configured such that they may be releasably fixed relative to each other such that they rotate in unison. Such screws are implanted by first fixing the leading and trailing elements and threadingly driving the screw into the proximal and distal bone fragments until the leading element is firmly embedded within the distal bone fragment and the trailing element is firmly embedded within the proximal bone fragment. The leading element is then unlocked from the trailing element and the leading element rotationally driven independent of the trailing element, tending to continue driving the leading element through the distal bone fragment. Given that the trailing element remains fixed in the proximal fragment, the rotation of the leading element results in the distal bone fragment being drawn back towards the proximal bone fragment, providing compression between the two bone fragments. With this arrangement, however, the second action, tending to continue driving the leading element through the distal fragment, may result in the leading element projecting from the distal surface of the distal bone fragment and reducing the degree of embedment of the leading element within the distal bone fragment. The typical coarse pitch thread, designed to cut into the bone material, may also result in failure in poor quality bone material when attempting to generate significant compression loads by driving the coarse steel thread further through the bone. The coarse thread also limits the degree of control of the amount of compression applied.

Document WO 2004/069031 A2 discloses a bone screw according to the preamble of claim 1.

### Object of the Invention

It is the object of the present invention to substantially overcome or at least ameliorate at least one of the above disadvantages.

### Summary of the Invention

In a first aspect, the present invention provides a compression bone screw comprising:
a shank having longitudinally opposing first and second shank portions, said first shank portion being externally threaded with a first shank thread;
a first element threadingly mounted on said first shank portion by way of a first element internal thread threadingly engaging said first shank thread, said first element being externally threaded with a first element external thread;
a second element mounted on, or integrally formed with, said second shank portion, said second element being externally threaded with a second element external thread, said second element external thread and said first element external thread being like-handed; and
said first shank portion and said first element being configured to operate in a first mode of operation in which said shank and said first element are rotationally driven in unison and a second mode of operation in which said shank is rotationally driven independent of said first element, such that said first element moves along said first shank portion towards said second element;
wherein a pitch of said first element external thread is substantially equal to a pitch of said second element external thread.

In a preferred form, said first element external thread and said second element external thread are each self-tapping threads.

In a preferred form, said second element external thread extends along substantially the entire length of said second element.

Typically, said pitch of said first and second element external threads is coarser than the pitch of said first shank thread.

In one or more preferred embodiments, said first shank portion is a trailing shank portion, said second shank portion is a leading shank portion, said first element is a trailing element and said second element is a leading element.

In a first embodiment, said second element is fixedly mounted on, or integrally formed with, said second shank portion.

In one or more embodiments, said first element external thread is opposite-handed to said first shank thread.

In one form, an end face of said first shank portion is provided with a primary drive formation and an end face of said first element is provided with a secondary drive formation, said primary and secondary drive formations being engageable with a drive tool in said first mode of operation for rotationally driving said shank and said first element in unison, said primary drive formation being engageable with the drive tool in said second mode of operation for rotationally driving said shank independently of said first element.

Typically, said primary drive formation is engageable with a primary drive head of the same drive tool in said first and second modes of operation, said secondary drive formation being engageable with a secondary drive head of the drive tool in said first mode of operation and disengageable from the secondary drive head in said second mode of operation. Alternatively, the primary drive head may be rotatable independently of the secondary drive head in the second mode of operation.

In one form, said secondary drive formation is in the form of a plurality of slots formed in an end face of said first element.

In one embodiment, in which said first element is a trailing element, said first element external thread has a first element external thread outer diameter that tapers towards a leading end of said first element.

In at least one embodiment, in which said first element is a trailing element, at least a leading region of said first shank thread tapers towards a trailing end of said shank and at least a leading region of said first element is configured to radially expand upon engagement with said leading region of said first shank thread. Typically, at least a leading region of said first element is provided with one or more longitudinally extending slits to enable radial expansion of said leading region of said first element.

Preferably, said compression bone screw further comprises a locking mechanism configured to lock said first element relative to said first shank portion upon installation of said compression bone screw.

In one form, said locking mechanism comprises a deformable detent secured to one of said first element and said first shank portion such that, upon installation of said compression bone screw, the other of said first element and said first shank portion engages and deforms said deformable detent to lock said first element to said first shank portion.

In one form, the locking mechanism comprises a locking device configured to engage said end face of said first shank portion and said end face of said first element to lock said first element relative to said first shank portion upon installation of said compression bone screw. Typically, said locking device engages said primary drive formation and said secondary drive formation.

In one form, said first shank portion and said first element are each configured to releasably engage a locking member to fix said first shank portion relative to said first element for said first mode of operation.

In such a form, typically said first shank portion is provided with a longitudinally extending shank groove and said first element is provided with a first element groove, said shank grove and said first element groove co-operating to receive said locking member in use, The locking member will typically be in the form of an elongate pin or screw.

In one or more embodiments:
said second shank portion is externally threaded with a second shank thread, said second shank thread being opposite-handed to said first shank thread, said second element being threadingly mounted on said second shank portion by way of a second element internal thread threadingly engaging said second shank thread; and
said second shank portion and said second element are configured to be rotationally driven in unison in said first mode of operation and such that said shank is rotationally driven independent of said second element in said second mode of operation such that said second element moves along said second shank portion towards said first element.

In one form, said shank, said first element and said second element are each configured to releasably engage a locking member to fix said shank relative to said first element and said second element for said first mode of operation.

In a preferred form, said locking member forms said locking mechanism.

In a preferred form, said shank is provided with a shank groove longitudinally extending along said first and second shank portions, said first element is provided with a first element groove, and said second element is provided with a second element groove, said shank groove, said first element groove and said second element groove co-operating to receive said locking member in use, said locking member being in the form of an elongate pin.

In at least one embodiment, first and second primary detents are provided at the end of said first and second shank portions respectively for engaging said first element and said second element respectively during said first mode of operation, thereby enabling said shank to be rotationally driven in unison with said first element and said second element in said first mode of operation, said second shank thread being opposite handed to said second element external thread.

In one embodiment, first and second secondary detents are provided between said first and second shank portions for engaging said first element and said second element respectively upon completion of said second mode of operation, thereby limiting movement of said first element towards said second element. In a preferred form, said first and second secondary detents are configured such that, upon engagement with said first and second elements respectively, said shank does not longitudinally extend beyond said first element or said second element.

In a second aspect, the present invention provides a compression bone screw comprising:
a shank having longitudinally opposing first and second shank portions, said first shank portion being externally threaded with a first shank thread, said second shank portion being externally threaded with a second shank thread, said second shank thread being opposite-handed to said first shank thread;
a first element threadingly mounted on said first shank portion by way of a first element internal thread threadingly engaging said first shank thread, said first element being externally threaded with a first element external thread; and
a second element threadingly mounted on said second shank portion by way of a second element internal thread threadingly engaging said second shank thread, said second element being externally threaded with a second element external thread, said second element external thread and said first element external thread being like-handed;
said shank, said first element and said second element being configured to operate in a first mode of operation in which said shank, said first element and said second element are rotationally driven in unison and a second mode of operation in which said shank is rotationally driven independent of said first element and said second element, such that said first element moves along said first shank portion towards said second element and said second element moves along said second shank portion towards said first element.

In one form, said first shank portion is a trailing shank portion, said second shank portion is a leading shank portion, said first element is a trailing element and said second element is a leading element.

In one specific embodiment, said first element external thread is configured to engage a mating thread of an aperture extending through a locking plate and said second element external thread is a self-tapping thread for engaging bone.

In one form, said shank, said first element and said second element are each configured to releasably engage a locking member to fix said shank relative to said first element and said second element for said first mode of operation.

In such a form, typically said shank is provided with a shank groove longitudinally extending along said first and second shank portions, said first element is provided with a first element groove, and said second element is provided with a second element groove, said shank groove, said first element groove and said second element groove co-operating to receive the locking member in use. The locking member will typically be in the form of an elongate pin or screw.

In one form first and second primary detents are provided at the end of said first and second shank portions respectively for engaging said first element and said second element respectively during said first mode of operation, thereby enabling said shank to be rotationally driven in unison with said first element and said second element in said first mode of operation, said second shank thread being opposite handed to said second element external thread.

In one form, first and second secondary detents are provided between said first and second shank portions for engaging said first element and said second element respectively upon completion of said second mode of operation, thereby limiting movement of said first element towards said second element.

Also described herein is a method of using the bone screw of the invention for fixing a proximal bone fragment to a distal bone fragment, said method comprising the steps of:
a) providing a compression bone screw comprising:
   (i) a shank having longitudinally opposing first and second shank portions, said first shank portion being externally threaded with a first shank thread;
   (ii) a first element threadingly mounted on said first shank portion by way of a first element internal thread threadingly engaging said first shank thread, said first element being externally threaded with a first element external thread; and
   (iii) a second element mounted on, or integrally formed with, said second shank portion, said second element being externally threaded with a second element external thread, said second element external thread and said first element external thread being like-handed;
b) drilling a hole through said proximal bone fragment into said distal bone fragment;
c) rotationally driving said screw into said hole with said second element leading, rotationally driving said first element, said second element and said shank in unison until said second element is embedded within said distal bone fragment and said first element is embedded in said proximal bone fragment;
d) rotationally driving said shank independently of said first element in a direction tending to draw said first and second elements together.

In one form, said second element is fixedly mounted on, or integrally formed with, said second shank portion, and in step (c) said shank is rotationally driven in unison with said second element.

In an alternate form, said second shank portion is externally threaded with a second shank thread, said second shank thread being opposite-handed to said first shank thread, said second element being threadingly mounted on said second shank portion by way of a second element internal thread threadingly engaging said second shank thread and, in step (c) said shank is rotationally driven independently of said second element.

Also described herein is a method of using the bone screw of the invention for fixing a proximal bone fragment to a distal bone fragment, said method comprising the steps of:
a) drilling a hole through said proximal bone fragment into said distal bone fragment;
b) securing a locking plate to an adjacent stable bone portion, aligning a threaded aperture extending through said locking plate with said hole;
c) providing a compression bone screw comprising:
   (i) a shank having longitudinally opposing first and second shank portions, said first shank portion being externally threaded with a first shank thread, said second shank portion being externally threaded with a second shank thread, said second shank thread being opposite-handed to said first shank thread;
   (ii) a first element threadingly mounted on said first shank portion by way of a first element internal thread threadingly engaging said first shank thread, said first element being externally threaded with a first element external thread matching the thread of said threaded aperture; and
   (iii) a second element threadingly mounted on said second shank portion by way of a second element internal thread threadingly engaging said second shank thread, said second element being externally threaded with a second element external thread, said second element external thread and said first element external thread being like-handed;
d) rotationally driving said screw through said threaded aperture into said hole with said second element leading, rotationally driving said first element, said second element and said shank in unison until said second element is embedded within said distal bone fragment and said first element is embedded within said threaded aperture;
e) rotationally driving said shank independently of said first element in a direction tending to draw said first and second elements together.

### Brief Description of the Drawings

Preferred embodiments of the present invention will now be described, by way of example only, with reference to the accompanying drawings wherein:
Figure 1 is a front elevation view of a compression bone screw according to a first embodiment;
Figure 2 is an end elevation view of the compression bone screw of Figure 1;
Figure 3 is an opposing end elevation view of the compression bone screw of Figure 1;
Figure 4 is an isometric view of the compression bone screw of Figure 1;
Figure 5 is a schematic cross-sectional view of the compression bone screw of Figure 1;
Figure 6 is a partially cross-sectioned view of a drive tool for use with the compression bone screw of Figure 1;
Figure 7 is a schematic view of a fractured bone with the compression bone screw of Figure 1 partly implanted;
Figure 8 is a schematic view of the fractured bone of Figure 7 with the compression screw of Figure 1 fully implanted;
Figure 9 is an isometric view of a compression bone screw according to a second embodiment;
Figure 10 is a further isometric view of the compression bone screw of Figure 9;
Figure 11 is a schematic cross-sectional view of the compression bone screw of Figure 9;
Figure 12 is an end elevation view of the compression bone screw of Figure 9;
Figure 13 is a front elevation view of the shank of the compression bone screw of Figure 9;
Figure 14 is a schematic view of a fractured bone with the compression bone screw of Figure 9 partly implanted;
Figure 15 is a schematic view of the fractured bone of Figure 14 with the compression bone screw of Figure 9 fully implanted;
Figure 16 is a front elevation of a compression bone screw according to a third embodiment;
Figure 17 is an isometric view of the compression bone screw of Figure 16;
Figure 18 is a further isometric view of the compression bone screw of Figure 16;
Figure 19 is a schematic cross-sectional view of the compression bone screw of Figure 16;
Figure 20 is a schematic cross-sectional view of the compression bone screw of Figure 16 in an implanted configuration;
Figure 21 is a schematic view of an ankle arthrodesis with the compression bone screw of Figure 16 partly implanted;
Figure 22 is a schematic view of the ankle arthrodesis of Figure 21 with the compression bone screw of Figure 16 fully implanted;
Figure 23 is a schematic perspective view of a trailing end portion of the compression bone screw of Figure 16;
Figure 24 is a perspective view of a locking device for use with the compression bone screw of Figure 16;

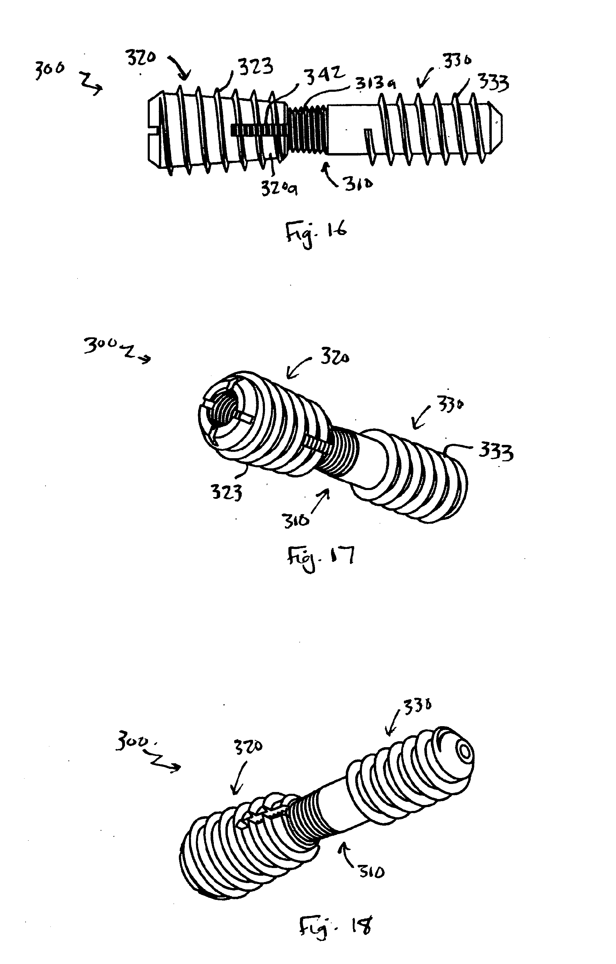

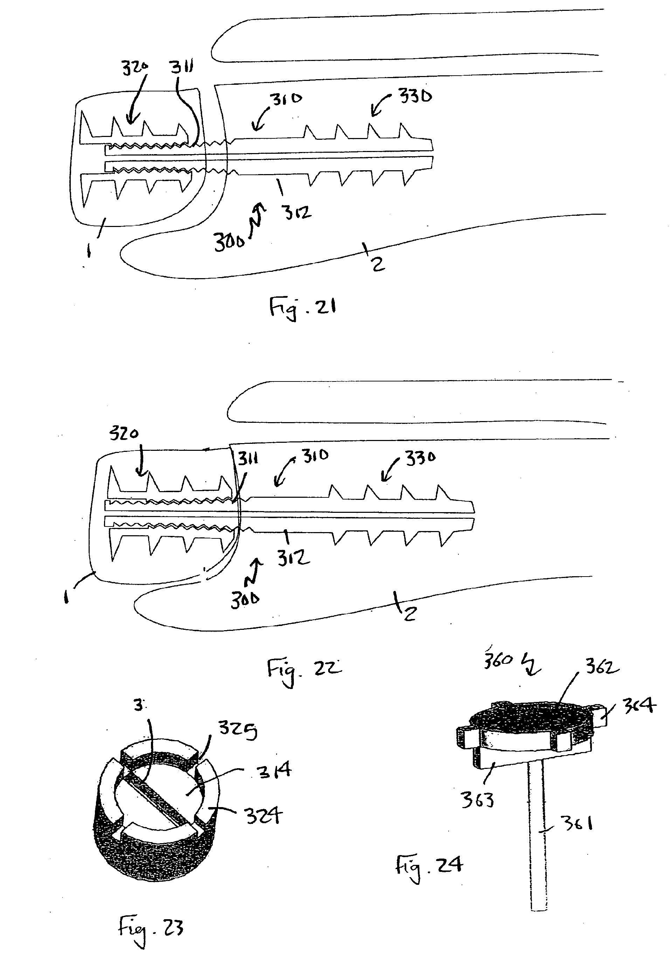

Figure 24 is a perspective view of a locking device for use with the compression bone screw of Figure 16;

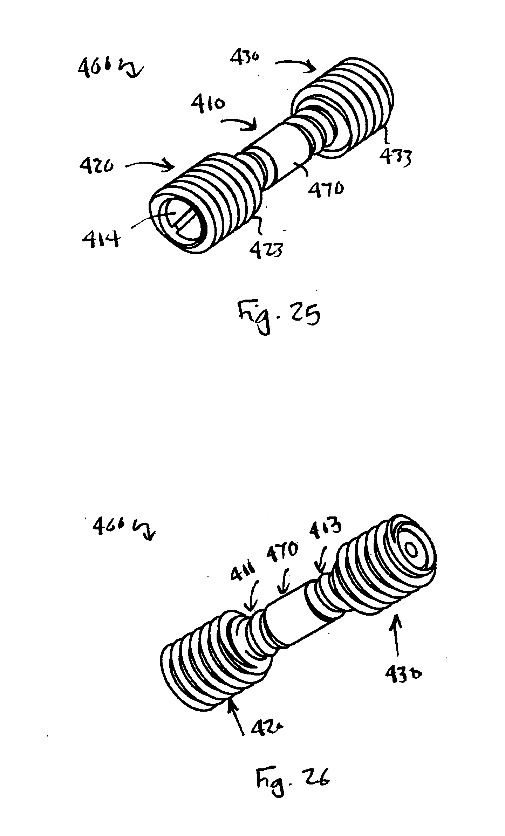

Figure 25 is a isometric view of a compression bone screw according to a fourth embodiment;
Figure 26 is a further isometric view of the compression bone screw of Figure 25;

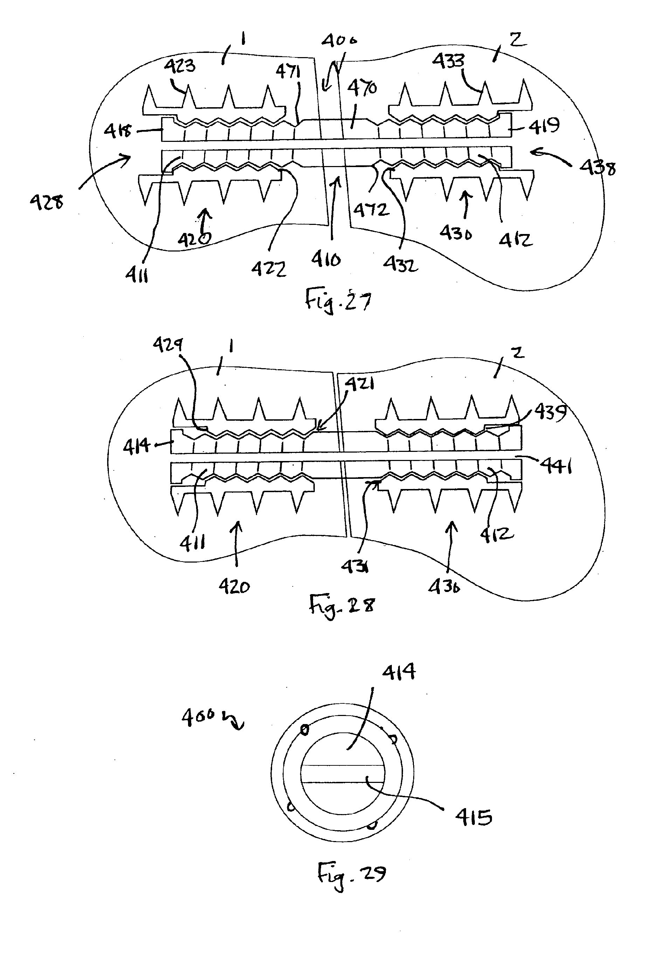

Figure 27 is a schematic view of a fractured bone with the compression bone screw of Figure 25 partly implanted;
Figure 28 is a schematic view of the fractured bone and compression bone screw of Figure 25 with the compression bone screw fully implanted;
Figure 29 is an end elevation view of the compression bone screw of Figure 25;

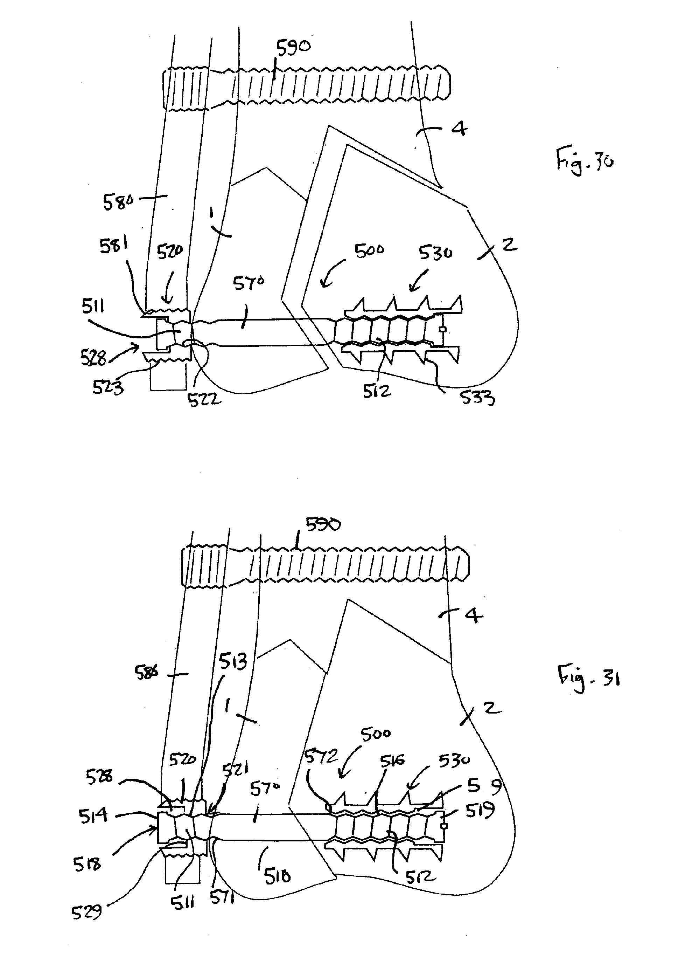

Figure 30 is a schematic view of a fractured bone with a compression bone screw according to a fifth embodiment partly implanted; and
Figure 31 is a schematic view of the fractured bone and compression bone screw of Figure 30 with the compression bone screw fully implanted.

### Detailed Description of the Preferred Embodiments

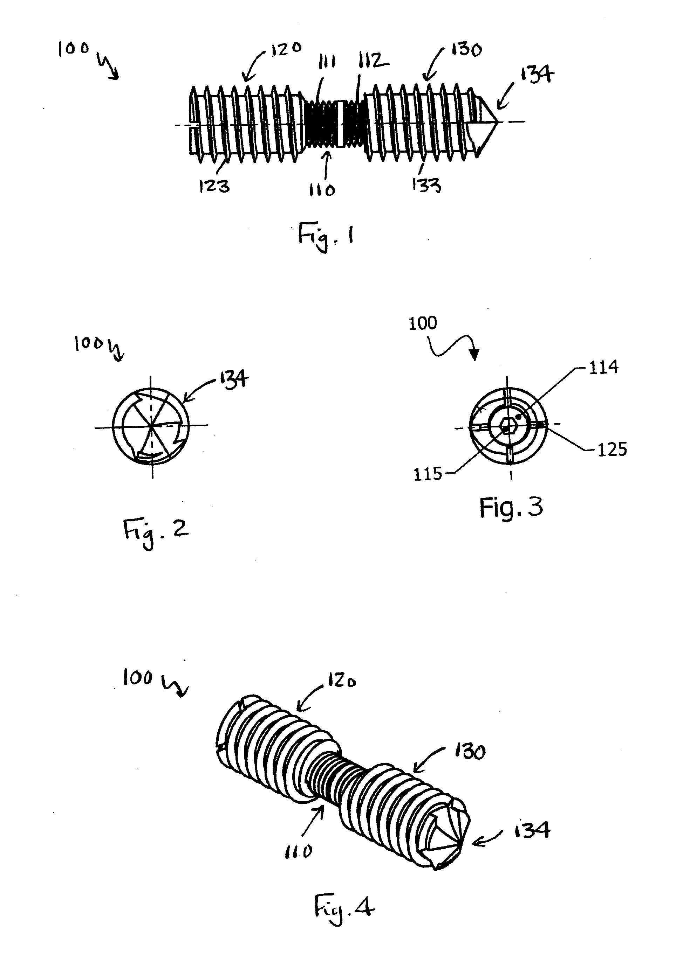

A compression bone screw 100 according to a first embodiment is depicted in Figures 1 to 5. The compression bone screw 100 comprises a shank 110, a first element 120 and a second element 130.

The shank 110 has longitudinally opposing first and second shank portions 111, 112. The first shank portion 111 is externally threaded with a first shank thread 113 that is here a left-handed thread. In the arrangement depicted, the shank 110 has an overall diameter of approximately 6 mm and the first shank thread 113 has a relatively fine pitch of approximately 1 mm.

The first element 120 has a first element aperture 121 longitudinally extending therethrough and a first element internal thread 122 formed on the wall of the first element aperture 121. The first element 120 is threadingly mounted on the first shank portion 111 by way of the first element internal thread 122 threadingly engaging the first shank thread 113. Accordingly, the first element internal thread 122 and first shank thread 113 are matching and are here in the form of machine threads. The first element 120 is also externally threaded with a first element external thread 123. The first element external thread 123 will typically extend along substantially the entire length of the first element 120. In the arrangement depicted, the first element external thread 123 is opposite-handed to the first shank thread 113, being a right-handed thread. The first element external thread 123 here has a relatively course pitch of approximately 2 mm, (compared to a relatively finer 1 mm pitch of the first shank thread 113), a thread depth of approximately 1.5 mm, and is self-tapping for screwing into bone material. In the arrangement depicted, the first element 120 typically has an overall diameter of approximately 11 mm and a length of the order of 15 mm.

In the first embodiment, the second element 130 is fixedly mounted on the second shank portion 112. The second element 130 could be fixed, typically by welding, to the end of the second shank portion 112 or alternatively the second shank portion 112 may project into a recess formed in the end of the second element 130 and be fixed thereto. It is also envisaged that the second element 130 might be integrally formed with the shank second portion 112, typically by machining, as a monoblock construction. The second element 130 is externally threaded with a second element external thread 133 that is like-handed to the first element external thread 123 (right-handed in the arrangement depicted) and an at least substantially identical pitch. The second element external thread 133 is again a self-tapping thread and will typically extend along substantially the entire length of the second element 130. The second element 130 has an equivalent length to that of the first element 120, being a length of approximately 15 mm and has a smaller overall diameter of approximately 10 mm, so that the first element external thread 123 is able to get a better purchase on the bone material after the second element external thread 133 has already passed therethrough. It is also envisaged, however, that the first and second elements 120, 130 may have the same overall diameter. It is further envisaged that the first and second elements 120, 130 may have differing lengths to suit applicability to different bones and clinical scenarios.

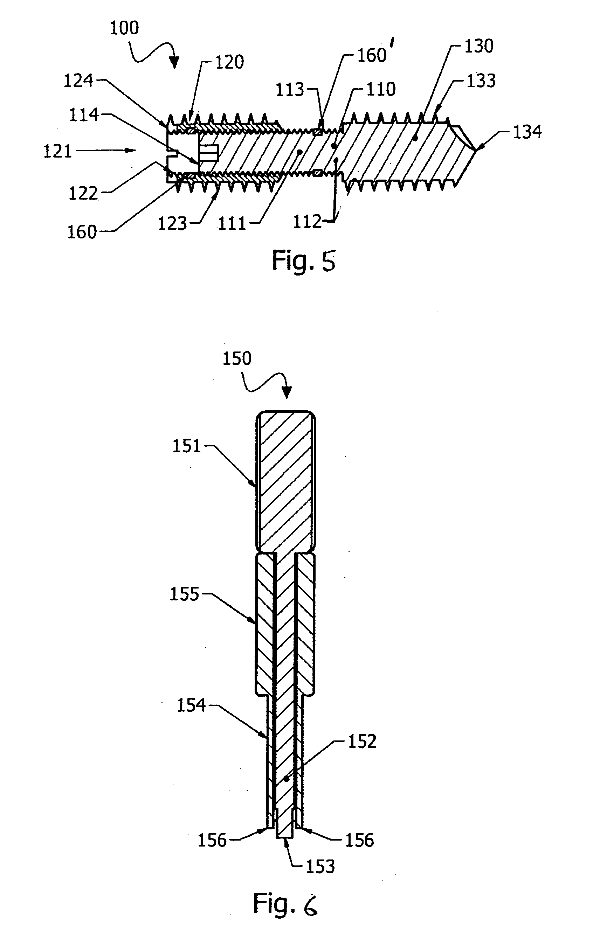

To protect against loosening of the compression bone screw 100 after implantation, the compression bone screw 100 here further comprises a locking mechanism configured to lock the first element 120 relative to the first shank portion 111 upon installation of the screw 100. In the first embodiment, the locking mechanism comprises a deformable detent 160 secured to the interior of the first element 120. As depicted in Figure 5, the detent 160 is here positioned adjacent the trailing first element end face 124 such that, as will be described below, upon installation of the compression bone screw 100 the first shank portion 111 engages the detent 160 and deforms the detent 160 so as to lock the first element 120 to the first shank portion 111. In the particular arrangement depicted, the deformable detent 160 is in the specific form of a collar insert which may be formed of nylon or another deformable material, in a similar manner to the nylon collar insert of a standard lock nut. Rather than being mounted internally on the first element 120, it is also envisaged that an equivalent deformable detent 160', again as depicted in Figure 5, could be mounted on the first shank portion 111, towards the second shank portion 112, so as to engage the first element 120 in a similar manner.

In the arrangement depicted, the second element 130 is the leading element of the compression bone screw 100 intended to lead when screwed into a fractured bone and, accordingly, is provided with a tapered self-tapping point 134 for drilling into bone material. The first element 120 is thus the trailing element, the first shank portion 111 is the trailing shank portion and the second shank portion 112 is the leading shank portion.

With the second element 130 being fixedly mounted on the second shank portion 112, the second element 130 rotates in unison with the shank 110 during use. The first shank portion 111 and first element 120 are configured to operate in a first mode of operation, as will be discussed below, in which the shank 110 and first element 120 are rotationally driven in unison and in a second mode of operation, as will again be discussed further below, in which the shank 110 is rotationally driven independent of the first element 120. In the first embodiment, these two modes of operation are provided for by way of primary and secondary drive formations provided on the first element 120 and the first shank portion 111 and an associated driving tool 150 depicted in Figure 6. The first shank portion 111 has a first shank end face 114 provided with a primary drive formation, here in the form of a hexagonal drive socket 115. The primary drive formation might, however, take any form of drive formation including a slot, Phillips head, star drive or other polygon drive form. The first element 120 has an annular trailing first element end face 124 that is provided with a secondary drive formation, here in the form of a plurality of slots 125, here configured as four equally spaced slots 125, formed in the first element end face 124. Again, the secondary drive formation may take any suitable drive formation form.

The drive tool 150 is formed with a handle 151 and elongate shaft 152 on the end of which is formed a hexagonal primary drive head 153 that is configured to engage the hexagonal drive socket 115 of the first shank portion 111. The drive tool 150 is further provided with a sleeve 154 that is mounted on the shaft 152, here so as to be longitudinally displaceable along the shaft 152 by way of finger grips 155 formed on the trailing end of the sleeve 154 for activation by a user gripping the handle 151. At the leading end of the sleeve 154 is provided a secondary drive head comprising four lugs 156 configured to engage the slots 125 provided in the first element end face 124. With the hexagonal primary drive head 153 engaging the hexagonal drive socket 115, the sleeve 154 may be advanced such that the lugs 156 engage the slots 125, for the first mode of operation to rotationally drive the first element 120 and shank 110 in unison. The sleeve 154 may be retracted, with the hexagonal primary drive head 153 still engaging the hexagonal drive socket 115, such that the lugs 156 disengage the slots 125, thereby allowing the shank 110 to be rotationally driven independently of the first element 120 in the second mode of operation. Alternatively, the sleeve 154 could be non-retractable and held stationary in engagement with the lugs 156 whilst the primary drive head 153 drives the shank 110 in the second mode of operation. Rather than providing the secondary drive head lugs 156 on the end of a retractable sleeve 154, it is also envisaged that two separate drive tools might be utilised, one having a drive head for engaging the primary drive formation only for the first mode of operation and the other having either a single drive head configured to engage both the primary and secondary drive formations, or separate drive heads for engaging each of the primary and secondary drive formations for the second mode of operation.

An alternate configuration is envisaged where the first element 120 forms the leading element and the second element 130 forms the trailing element. In such a configuration however, the first element 120, being the element threadingly mounted on the shank 110, would not be as accessible for access by a drive tool for driving the first element 120 in unison with the first shank portion 111. As an alternative, the first element 120 could be releasably fixed to the first shank portion 111 for the first mode of operation in a similar manner to that described below in relation to the second embodiment.

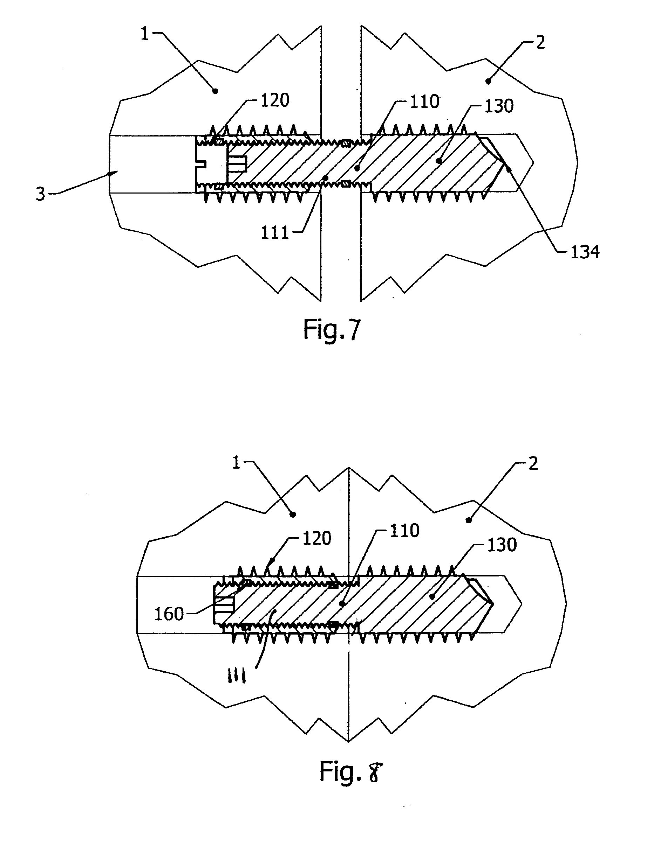

The fixing of proximal and distal bone fragments 1, 2 of a fractured bone using the compression bone screw 100 of the first embodiment will now be described with reference to Figures 7 and 8.

With the proximal and distal bone fragments 1, 2 appropriately positioned, a guide hole 3 with a diameter of approximately 8 mm for the arrangement depicted is first drilled through both the proximal and distal bone fragments 1, 2. The guide hole 3 may extend through the thickness of the distal fragment 2, or may be a blind hole as depicted. The guide hole 3 may then be pre-tapped, particularly if the bone material is particularly hard or if the first and second element external threads 123, 133 are not of a self-tapping configuration. The tapered point 134 of the second element 130 is then inserted into the guide hole 3 formed in the proximal bone fragment 1. The drive tool 150 is engaged with the compression bone screw 100, engaging the hexagonal primary drive head 153 with the hexagonal drive socket 115 formed in the end face 114 of the first shank portion 111 and the sleeve 154 of the drive tool 150 is advanced such that the lugs 156 engage the slots 125 formed in the end face 124 of the first element 120. The drive tool 150 is then rotationally driven by hand in a clockwise direction in the first mode of operation, thereby rotationally driving the first element 120 in unison with the shank 110 (and second element 130), advancing the entire compression bone screw 100 through the guide hole 3. With the pitch of the first element external thread 123 and second element external thread 133 being at least substantially identical, the first and second elements 120, 130, rotating in unison, will advance through the proximal and distal bone fragments 1, 2 respectively at at least a substantially equal rate so that the first mode of operation will not tend to either draw the proximal and distal bone fragments 1, 2 towards each either or drive them apart. The compression bone screw 100 is rotationally driven until the second element 130 is firmly embedded within the distal bone fragment 2 and the first element 120 is firmly embedded within the proximal bone fragment 1.

At this point, the proximal and distal bone fragments 1, 2 will be firmly fixed in relation to each other, but without any compression being applied at the interface between the bone fragments 1, 2. The sleeve 154 is then retracted so as to disengage the lugs 156 from the slots 125 (or kept engaged with the lugs 156 but held stationary). The drive tool 150 is then further rotationally driven in the clockwise direction in the second mode of operation (or at least the shaft 152 rotationally driven). This results in rotation of the shank 110 together with the second element 130, independently of the first element 120 which remains stationary within its embedded position within the proximal bone fragment 1. The rotation of the second element 130 tends to advance the second element 130 deeper into the distal bone fragment 2 by virtue of the right-handed thread of the second element external thread 133, thereby effectively drawing the distal bone fragment 2 back toward the proximal bone fragment 1. At the same time, rotation of the shank 110 tends to retract the first shank portion 111 relative to the first element 120 by virtue of the opposite left-handed thread of the first shank thread 113. The first element 120 thus moves forward along the first shank portion 111 towards the second element 130. This draws the proximal bone fragment 1 toward the distal bone fragment 2, generating the desired compression between the bone fragments 1, 2, without displacement of the first element 120 within the proximal bone fragment 1. Having the first shank thread 113 and second element external thread 133 opposite-handed ensures that the relative motion of the proximal and distal bone fragments 1, 2 towards each other in a single revolution of the shank 110 is equal to the sum of the pitches of the two threads. Accordingly, a desired level of compression can be achieved through significantly less revolutions than is required with compression bone screws that rely on unequal pitched threads to achieve compression. Both the first and second element external threads 123, 133 being made with substantially the same coarse pitch, provide improved fixation in cancellous bone.

When the first shank portion 111 is drawn sufficiently back into the first element 120 during the second mode of operation, the first shank portion 111 engages the deformable detent 160 to lock the first element 120 onto the first shank portion 111, thereby inhibiting loosening of the compression bone screw 100 after implementation which might otherwise result from micro-movement of the proximal and distal bone fragments 1, 2 during the healing process.

The implantation of the compression bone screw 100 may be conducted with the assistance of a guide wire extended through the guide hole 3. Where a guide wire is to be utilised, a bore hole (not depicted) will extend through the centre of the shank 110 and the second element 130 for receipt of the guide wire 3. Similarly, a bore hole will extend through the centre of the drive tool 150. The drive tool 150 and compression bone screw 100 will thus be mounted on the guide wire throughout the implantation process.

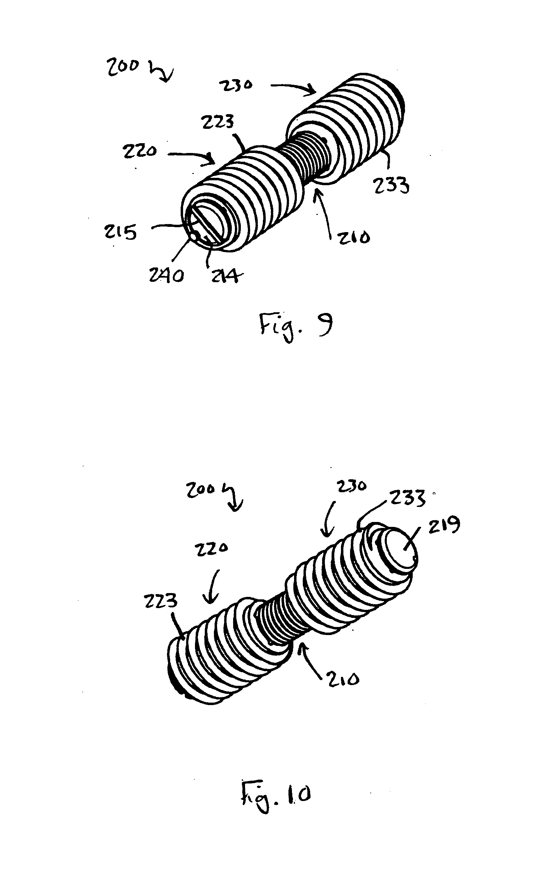

A compression bone screw 200 according to a second embodiment is depicted in Figure 9 through 13. The compression bone screw 200 again comprises a shank 210, first element 220 and second element 230. Those features of the compression bone screw 200 of the second embodiment that are identical, or equivalent, to features of the compression bone screw 100 of the first embodiment are provided with like reference numerals, increased by 100. The shank 210, first element 220 and second element 230 may again be formed of stainless steel or any other suitable biologically inert engineering material, including a bioabsorbable material such as polylactic acid. In the arrangement depicted, the second element 230 is the leading element of the compression bone screw 200 intended to lead when screwed into a fractured bone. The first element 220 is the trailing element, the first shank portion 211 is the trailing shank portion and the second shank portion 212 is the leading shank portion.

The shank 210 again has longitudinally opposing first and second shank portions 211, 212. The first shank portion is externally threaded with a first shank thread 213 whilst the second shank portion 212 is externally threaded with a second shank thread 216. The first and second shank threads 213, 216 are opposite handed. In the arrangement depicted, the first shank thread 213 is a left-handed thread whereas the second shank thread 216 is a right-handed thread, although it is envisaged that the first shank thread 213 is a right-handed thread and the second shank thread 216 is a left-handed thread (although this would require rotational driving of the shank in a counter-clockwise direction during the second mode of operation rather than clockwise as discussed below). In the arrangement depicted, the shank 210 again has an overall diameter of approximately 6 mm and the first and second shank threads 213, 216 have a pitch of approximately 1 mm.

The first element 220 has a first element aperture 221 longitudinally extending therethrough and a first element internal thread 222 formed on the wall of the first element aperture 221. The first element 220 is threadingly mounted on the first shank portion 211 by way of the first element internal thread 222 threadingly engaging the first shank thread 213. The first element internal thread 222 and first shank thread 213 are matching and are here in the form of machine threads. The first element 220 is also externally threaded with a first element external thread 223. The first element external thread 223 will typically extend along substantially the entire length of the first element 220. In the arrangement depicted, the first element external thread 223 is opposite-handed to the first shank thread 213, being a right-handed thread. The first element external thread 223 is here again a self-tapping thread having a relatively coarse pitch of approximately 2 mm and depth of approximately 1.5 mm. In the arrangement depicted, the first element 220 typically has an overall diameter of approximately 11 mm and a length of the order of 15 mm.

The second element 230, rather than being fixed to the shank 210 as is the case in the first embodiment, is threadingly mounted on the second shank portion 212. The second element 230 has a second element aperture 231 longitudinally extending therethrough and a second element internal thread 232 formed on the wall of the second element aperture 231. The second element 230 is threadingly mounted on the second shank portion 212 by way of the second element internal thread 232 threadingly engaging the second shank thread 216. The second element internal thread 232 and second shank thread 216 are matching and are here in the form of machine threads. The second element 230 is also externally threaded with a second element external thread 233. The second element external thread 230 will typically extend along substantially the entire length of the second element 230. The second element external thread 233 is again like-handed to the first element external thread 223, being a right-handed thread. The second element external thread 233 is again a self-tapping thread having a relatively coarse pitch of approximately 2 mm (substantially identical to the pitch of the first element external thread 223) and depth of approximately 1.5 mm for reliable purchase in cancellous bone material. The second element 233 has an equivalent length to that of the first element 220, being a length of approximately 15 mm and an equivalent overall diameter of approximately 11 mm. It is further envisaged that the first and second elements 220, 230 may have differing lengths to suit applicability to different bones and clinical scenarios and that the second element external thread 233 may have a coarser pitch, which may be a significantly coarser pitch, than the first element external thread 223.

So as to retain the first and second elements 220, 230 on the shank 210, a circlip, thread end, weld, enlarged head or other form of first and second primary detents 218, 219 may be fitted to, or formed at, the end of each of the first and second shank portions 211,212 respectively. The shank 210, first element 220 and second element 230 are configured to operate in a first mode of operation, as will be discussed below, in which the shank 210, first element 220 and second element 230 are rotationally driven in unison and in a second mode of operation, as will again be discussed further below, in which the shank 210 is rotationally driven independent of the first and second elements 220, 230. Rather than using drive formations on each of the first and second elements 220, 230 and shank 210 as with the first embodiment, these two modes of operation are provided for by releasably locking or fixing the first and second elements 220, 230 to the shank 210. This is done by configuring the first shank portion 211, first element 220, second shank portion 212 and second element 230 to releasably engage a locking member to lock them relative to each other for the first mode of operation.

Specifically, in the arrangement depicted, the shank 210 is provided with a shank groove 217 longitudinally extending along its length. The first and second elements 220, 230 are provided with a longitudinally extending first element groove 227 and a second element groove 237 respectively. The shank groove 217, first element groove 227 and second element groove 237 co-operate to receive a locking member in the form of a locking pin 240. In the arrangement depicted, the locking pin 240 is generally cylindrical in form with a diameter of approximately 2.0 mm. The shank groove 217, first element groove 227 and second element groove 237 are each being semi-circular in cross-section so as to neatly receive the locking pin 240. The locking pin 240 may, however, be of any suitable cross-section. The locking pin 240 may be inserted in the aperture defined by the shank groove 217, first element groove 227 and second element groove 237 to fix the shank 210, first element 220 and second element 230 for rotationally driving the compression bone screw 200 in the first mode of operation. The locking pin 240 may be removed such that the shank 210 may be rotationally driven independently of the first and second elements 220, 230.

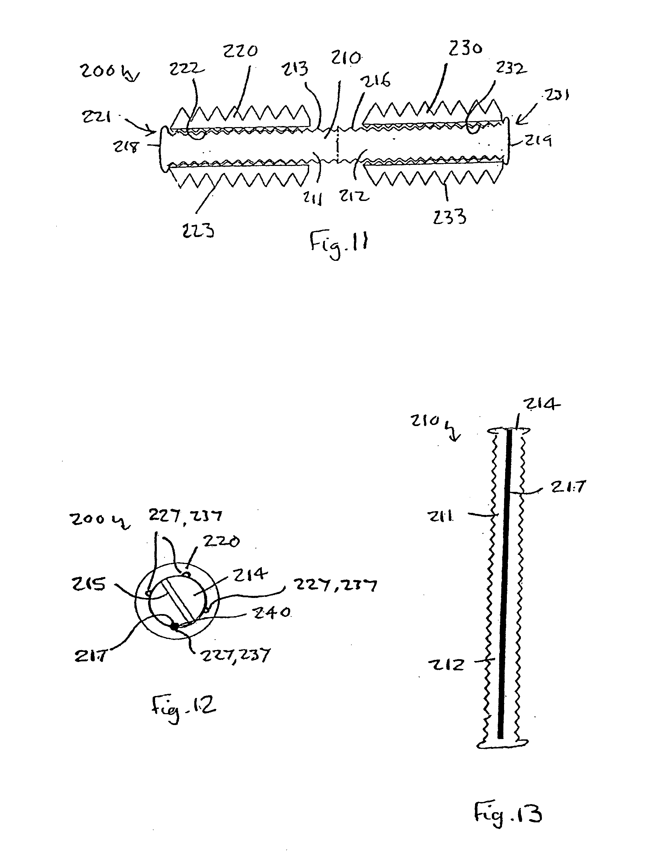

In the particular arrangement depicted in Figure 12, the first and second elements 220, 230 are provided with a plurality of first elements grooves 227 and second element grooves 237 respectively, with the grooves being spaced about the inner periphery of the first and second elements 220, 230. Provision of multiple first and second element grooves 227, 237 provide some flexibility of alignment of the shank groove 217 with the first and second element grooves 227, 237 to allow insertion of the locking pin 240. This is particularly beneficial in enabling re-insertion of the locking pin 240 after implantation so as to act as a locking mechanism locking the first and second elements 220, 230 relative to the shank 210 after installation to prevent loosening of the compression bone screw 200. Rather than re-inserting the locking pin 240, the compression bone screw 200 could be provided with deformable detents associated with each of the first and second elements 220, 230 in the same manner as the deformable detents 160, 160' described above in relation to the first embodiment.

The first shank portion 211 has a first shank end face 214 that is provided with a primary drive formation, here in the form of a drive slot 215, for rotationally driving the shank 210 in both the first and second modes of operation. The primary drive formation may again take any suitable form including a hex socket or star drive.

It is envisaged that the locking member arrangement described above may equally be applied to the compression bone screw 100 of the first embodiment, with grooves provided on the first shank portion 111 and first element 120 only for receipt of a shorter locking pin to fix the first element 120 to the shank 110 for the first mode of operation. Such a shorter locking pin could also be utilised as a locking mechanism to lock the first element 120 in relation to the first shank portion 111 after installation of the compression bone screw 100 of the first embodiment and, to assist in this regard, multiple grooves may again be provided on the first element 120 in a similar manner to that described above. It is also envisaged that the compression bone screw 200 of the second embodiment may be configured similar to the compression bone screw 100 of the first embodiment, providing drive formations on each of the first and second elements 220, 230 and shank 210, with the drive formation on the shank 210 only being engaged by a driving tool during the second mode of operation and the drive formations of the shank 210 and first and second elements 220, 230 being engaged by a drive tool for the first mode of operation.

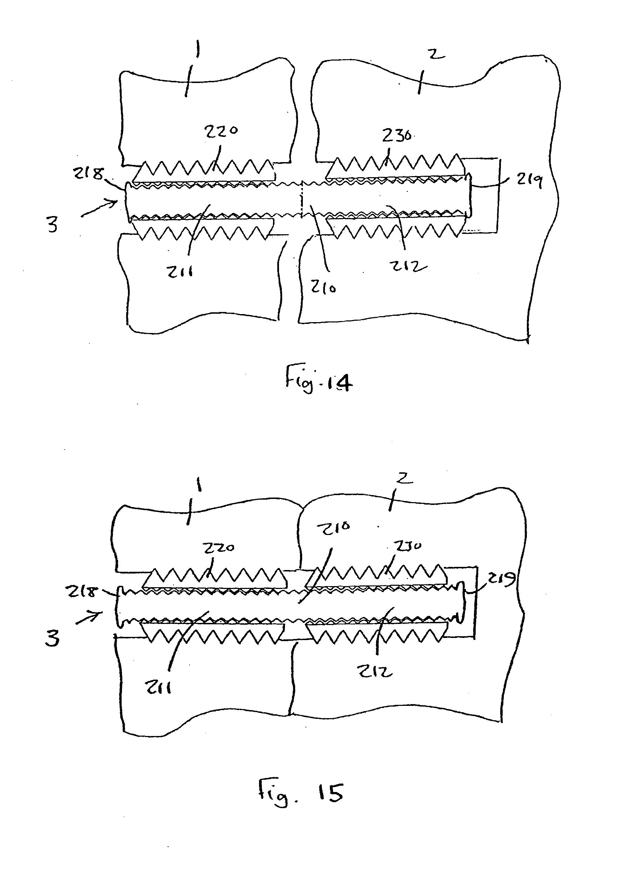

The fixing of proximal and distal bone fragments 1, 2 of a fractured bone using the compression bone screw 200 of the second embodiment will now be described with reference to Figures 14 and 15.

As with the first embodiment, with the proximal and distal bone fragments 1, 2 appropriately positioned, a guide hole 3 with a diameter of approximately 8 mm (for the arrangement depicted) is first drilled through the proximal and distal bone fragments 1, 2. The locking pin 240 is inserted in the aperture defined by the shank groove 217, first element groove 227 and second element groove 237 to fix the first and second elements 220, 230 to the shank 210. The compression bone screw 200 is inserted into the guide hole 3 formed in the proximal bone fragment 1 with the second element 230 leading. A drive tool in the form of a standard screwdriver is engaged with the compression bone screw 200, engaging the drive slot 215 formed in the first shank end face 214. The drive tool is then rotationally driven by hand in a clockwise direction in the first mode of operation, thereby rotationally driving the first element 220 and second element 230 in unison with the shank 210, advancing the entire compression bone screw 200 through the guide hole 3. As with the first embodiment, with the pitch of the first element external thread 223 and second element external thread 233 being at least substantially identical, the first and second elements 220, 230, rotating in unison, will advance through the proximal and distal bone fragments 1, 2 respectively at at least a substantially equal rate so that the first mode of operation will not tend to either draw the proximal and distal bone fragments 1, 2 towards each either or drive them apart. As noted above in relation to the first embodiment, however, it is envisaged that the second element external thread 233 may be coarser than the first element external thread 223 such that the proximal and distal bone fragments will be drawn closer together during the first mode of operation. The compression bone screw 200 is rotationally driven until the second element 230 is firmly embedded within the distal bone fragment 2 and the first element 220 is firmly embedded within the proximal bone fragment 1.

At this point, the proximal and distal bone fragments 1, 2 will be firmly fixed in relation to each other, but without any compression being applied at the interface between the bone fragments 1, 2. The locking pin 240 is then removed so as to unlock the first and second elements 220, 230 from the shank 210. The drive tool is then further rotationally driven in the clockwise direction in the second mode of operation. This results in rotation of the shank 210 independently of the first and second elements 220, 230 which remain stationary within their embedded positions within the proximal and distal bone fragments 1, 2 respectively. The left-handed configuration of the first shank thread 213 tends to advance the first element 220 along the shank 210, thereby advancing the proximal bone fragment 1 towards the distal bone fragment 2. At the same time, the right-handed configuration of the second shank thread 216 tends to move the second element 230 back along the shank 210, thereby drawing the distal bone fragment 2 back towards the proximal bone fragment 1. Accordingly, the proximal and distal bone fragments 1, 2 are drawn towards each other, generating the desired compression between the bone fragments 1, 2 without displacement of the first element 220 within the proximal bone fragment 1 or displacement of the second element 230 within the distal bone fragment 2. The degree of compression generated can be accurately controlled by utilising a torque wrench on the drive tool, as there will be a direct correlation between torque applied to the shank 210 with compression generated between the proximal and distal bone fragments 1, 2. Again, following implantation, the locking pin may be re-inserted, making fine adjustments to the position of the shank 210 in relation to the first and second elements 220, 230 as required to align the shank groove 217 with one of the first element grooves 227 and one of the second element grooves 237, thereby locking the first and second elements 220, 230 relative to the shank 210 to prevent loosening of the compression bone screw 200.

Implantation of the compression bone screw 200 may again be conducted with the assistance of a guide wire extended through the guide hole 3, with bore holes extending through the centre of the shank 210 and the drive tool.

In an alternate form of the compression bone screw 200 of the second embodiment, rather than forming the first shank thread 213 in a left-handed configuration and the second shank thread 216 in a right-handed configuration, it is envisaged that the first shank thread 213 may be of a right-handed configuration and the second shank thread 216 in a left-handed configuration such that the first shank thread 213 is like-handed to the first and second element external threads 223, 233. In such a configuration, however, the shank 210 would need to be rotationally driven in a counter-clockwise direction in the second mode of operation. With such a configuration, the detents 218, 219 provided at the end of each of the first and second shank portions 211, 212 may be utilised to fix the first and second elements 220, 230 to the first and second shank portions 211, 212 respectively during the first mode of operation. In this arrangement, the first and second elements 220, 230 would be located on the first and second shank portions 211, 212 abutting against the detents 218, 219 prior to implantation of the compression bone screw 200. Rotation of the shank 210 in the clockwise direction will tend to drive the first and second elements 220, 230 towards the ends of the shank 210, however, the detents 218, 219 at the end of each of the first and second shank portions 211, 212 will prevent the first and second elements 220, 230 from advancing towards the ends, thereby effectively fixing the first and second elements 220, 230 in relation to the shank 210 during the first mode of operation. Accordingly, the locking pin 240 need not be utilised. In the second mode of operation, rotation of the shank 210 in the counter-clockwise direction will draw the first and second elements 220, 230 towards each other along the first and second shank portions 211, 212 in the same manner as described above. It is envisaged, however, that the locking pin 240 may still be utilised in the manner described above if subsequent removal of the compression bone screw 200 is required, aligning the shank groove 217, first element groove 227 and second element groove 237 and inserting the locking pin 240 prior to rotationally driving the shank 210 in the counter-clockwise direction to withdraw the compression bone screw 200.

The same principle may also be utilised in the first embodiment to effectively lock the first element 120 to the first shank portion 121 in the second mode of operation.

It is further envisaged that, in both embodiments described above, the first and second element external threads may be left-handed rather than right-handed, counter-clockwise driving of the drive tool would again be required and, in the first embodiment, the first shank thread would be right-handed.

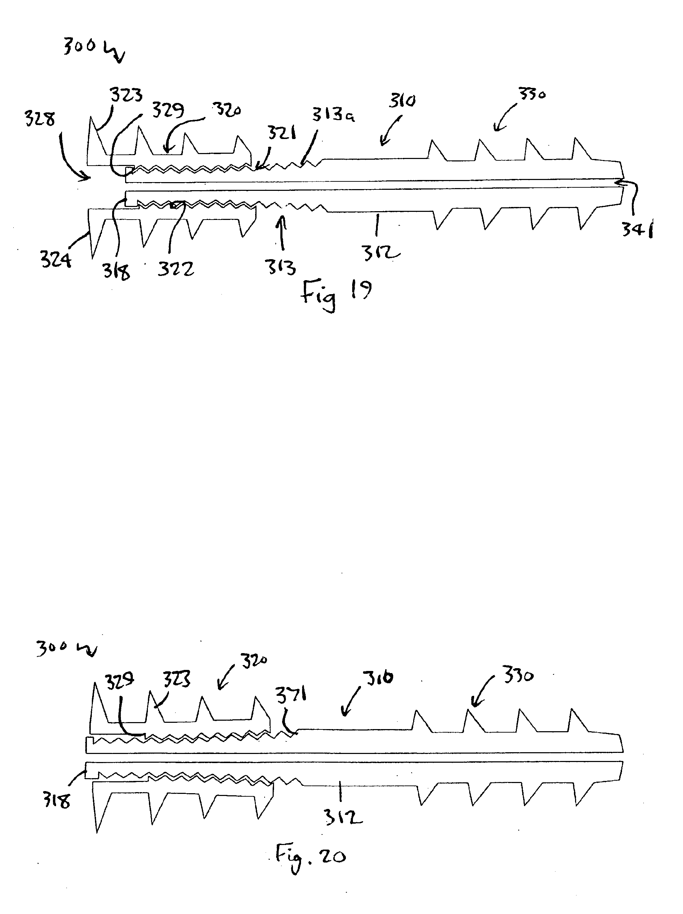

A compression bone screw 300 according to a third embodiment is depicted in Figures 16 to 23. The compression bone screw 300 is a modified version of the compression bone screw 100 of the first embodiment and shares the same basic configuration. Those features of the compression bone screw 300 of the third embodiment that are identical, or equivalent, to features of the compression bone screw 100 of the first embodiment are again provided with like reference numerals, here increased by 200. The compression bone screw 300 comprises a shank 310, a first element 320 and a second element 330. The shank 310, first element 320 and second element 330 may be formed of the same materials as per the first and second embodiments. In the arrangement depicted, the first element 320 is the trailing element, the first shank portion 311 is the trailing shank portion and the second shank portion 312 is the leading shank portion.

The shank 310 has longitudinally opposing first and second shank portions 311, 312. The first shank portion 311 is externally threaded with a first shank thread 313 that is again a left-handed thread. A leading region 313a of the first shank thread 313 is tapered, with the first shank thread 313 tapering in this region towards the first shank end face 314 at a taper angle that will typically be about 2 to 3 degrees. The taper angle may, however be larger, with angles up to at least 30 degrees being envisaged. Here the first shank thread 313 has an overall diameter of approximately 4.0 mm at its leading end adjacent the second shank portion 312, and an overall diameter of approximately 3.4 mm along the length of the trailing region of the first shank thread 313. As with the first embodiment, the first shank thread 313 here has a pitch of approximately 1 mm. The second shank portion 312 is here of a cylindrical non-threaded form. A central bore hole 341 sized to receive a guide wire is provided along the length of the shank 310, as would typically be the case with the compression bone screw 100 and 200 of the first and second embodiments (although not depicted). A first primary detent 318, in the form of an enlarged head, is formed at the trailing first shank end face 314.

The first element 320 has a first element aperture 321 longitudinally extending therethrough and a first element internal thread 322 formed on the wall of the first element aperture 321. The first element 320 is threadingly mounted on the first shank portion 311 by way of the first element internal thread 322 threadingly engaging the first shank thread 313, as per the first embodiment. The first element 320 is externally threaded with a first element external thread 323 which again extends along substantially the entire length of the first element 320. In this embodiment, the first element external thread 323 is opposite-handed to the first shank thread 313, being a right-handed thread. The first element external thread 323 here has a relatively coarse pitch of approximately 2 mm and an overall diameter that tapers towards a leading end of the first element 320. To achieve this tapering of the first element thread overall diameter, the thread depth decreases towards the leading end of the first element 320, here decreasing from approximately 2.8 mm to approximately 1.5 mm. The first element thread 323 is also self-tapping for screwing into bone material. The first element 320 typically has an overall diameter of approximately 10.5 mm its trailing end and a length of the order of 15 mm. The trailing first element end face 314 is provided with a first element recess 328 for receipt of the first primary detent 318. The base of the first element recess 328 forms an annular first element shoulder 329 which engages the first primary detent 318 at one end of the extent of travel of the first element 320 along the first shank portion 311, to retain the first element 320 on the first shank portion 311. Travel of the first element 320 along the first shank portion 311 in the opposing direction is limited by engagement of the leading end of the first element 320 with a first secondary detent 371 defined at the junction between the threaded first shank portion 311 and the non-threaded second shank portion 312. The first secondary detent is effectively defined by the runout of the first shank thread 313. The first element recess 328 has a length such that the first primary detent 318 does not project beyond the first element recess 328 at any point along the extent of travel of the first element 320, ensuring that there is no overhang of the shank 310 from the first element 320 upon implantation.

Referring specifically to Figure 16, the first element 320 is provided with one or more longitudinally extending slits 342 extending from the leading end of the first element 320, along about 30 to 50 percent of the length of the first element, to enable radial expansion of the leading region 320a of the first element 320 as it is advanced along the tapered leading region 313a of the first shank thread 313 during the second mode of operation of the compression bone screw 300 as will be discussed below.

The second element 330 is fixed in relation to the shank 310, here specifically being integrally formed with the shank 310 such that the second element 330 and shank rotate in unison during use. The second element 330 is of the same general configuration as the second element 130 of the compression bone screw 100 of the first embodiment, being externally threaded with a second element external thread 333 that is like-handed to the first element external thread 323 (right-handed in the arrangement depicted) and which extends along substantially the entire length of the second element 330. The second element external thread 333 again typically has a substantially identical pitch to that of the first element external thread 323 and has a diameter slightly less than that of the leading end of the first element 320, so that the first element external thread 323 is able to get a better purchase on the bone material after the second element external thread 333 has already passed therethrough.

Again as with the first embodiment, the first shank portion 311 and first element 320 are configured to operate in a first mode of operation in which the shank 310 and first element 320 are rotationally driven in unison and in a second mode of operation in which the shank 310 is rotationally driven independent of the first element 320. Again similar to the first embodiment, these two modes of operation are provided for by way of primary and secondary drive formations formed on the first element 320 and the first shank portion 311 respectively and an associated driving tool equivalent to that of the driving tool 150 depicted in Figure 6. The first shank end face 314 is provided with a primary drive formation, shown in Figure 23 in the form of a slot 315, for engaging a primary drive head in the form of a blade rather than the hexagonal drive head 153 of the drive tool 150. Again, any suitable form of primary drive formation might be provided on the first shank end face 314. The annular trailing first element end face 324 is provided with a secondary drive formation in the form of a plurality of slots 325 as per the first embodiment.

The configuration of the first element 320 and first shank portion 311 of the third embodiment may also be applied to the first element 220 and first shank portion 211 of the compression bone screw 200 of the second embodiment, particularly in relation to the tapering aspects of the first element thread 323 and tapered leading region 313a of the first shank thread 313 along with the slits 342 in the first element 320.

The procedure for fixing proximal and distal bone fragments 1, 2 of a fractured bone using the compression bone screw 300 of the third embodiment is generally as per the above described procedure in relation to the compression bone screw 100 of the first embodiment, and will now be described in further detail with reference to Figures 21 and 22.

As with the first embodiment, a guide hole 3 is first drilled through the proximal and distal bone fragments 1, 2 and the tapered self-tapping point 334 of the second element 330 inserted into the guide hole 3 formed in the proximal bone fragment 1. The drive tool 150 is engaged with the compression bone screw 300 engaging the primary slot 315 and secondary drive formation slots 325 and the drive tool 150 rotationally driven in a clockwise direction in the first mode of operation, thereby rotationally driving the first element 320 in unison with the shank 310 and second element 330, advancing the entire compression bone screw 300 through the guide hole 3. As with the procedure for the first embodiment, once the second element 330 is firmly embedded within the distal bone fragment 2 and the first element 320 is firmly embedded within the proximal bone fragment 1, the sleeve 154 of the drive tool 150 is retracted so as to disengage the lugs 156 from the secondary drive formation slots 125 and the drive tool is further rotationally driven in a clockwise direction in the second mode of operation. This results in rotation of the shank 310 together with the second element 330, independent of the first element 320 which remains stationary. Rotation of the second element 330 tends to advance the second element 330 deeper into the distal bone fragment 2, thereby effectively drawing the distal bone fragment 2 back towards the proximal bone fragment 1. At the same time, rotation of the shank 310 tends to retract the first shank portion 311 relative to the first element 320. The first element 320 thus moves forward along the first shank portion 311 towards the second element 330. This draws the proximal bone fragment 1 towards the distal bone fragment 2, generating the desired compression between the bone fragments 1, 2 without displacement of the first element 320 with any proximal bone fragments.

At the same time, as the first element is drawn along the first shank portion 311, engaging the tapered leading region 313a of the first shank thread 313, the leading region 320a of the first element 320 radially expands, by expansion of the slits 342. This radial expansion provides a compressive preload between the first element 320 and bone increasing the pullout strength of the compression bone screw 300 in weaker, particularly osteoporotic, bone, thereby increasing the strength of the overall construct. A similar preload is applied in the trailing region of the first element 320 by virtue of the taper of the first element thread 323 as the first element 320 is advanced through the proximal bone fragment 1 during the first mode of operation. A similar effect may be achieved with the compression bone screw 200 of the second embodiment utilising a tapered first shank thread and slitted and tapered first element.

Once implantation of the compression bone screw 300 is complete, when the desired compression is achieved, the first element 320 may be locked in relation to the shank 311 by way of a locking mechanism in the form of a locking device 360 as depicted in Figure 24. The locking device 360 has an elongate shaft 361 configured to extend into the bore hole 341 in the shank 310. The locking device also has a head 362, the leading face of which is provided with a primary locking structure, here in the form of an elongate rib 363, configured to engage the first shank end face 314, particularly the primary drive formation slot 315 of the first shank portion 311. The locking device body 362 is further provided with a secondary locking structure, here in the form of four radially projecting lugs 364, configured to engage the first element end face 324, particularly the secondary drive formation slots 325. Once in place, the locking device 360 prevents any relative rotation between the first element 320 and the first shank portion 311. An equivalent locking device may also be used with the compression bone screw 100 of the first embodiment.

A compression bone screw 400 according to a fourth embodiment is depicted in Figures 25 through 29. The compression bone screw 400 is a modified version of the alternate form of the compression bone screw 200 of the second embodiment as described above and shares the same basic configuration. Those features of the compression bone screw 400 of the fourth embodiment that are identical, or equivalent, to features of the compression bone screw 200 of the second embodiment are again provided with like reference numerals, here increased by 200. The compression bone screw 400 comprises a shank 410, a first element 420 and a second element 430. In the arrangement depicted, the first element 420 is the trailing element, the first shank portion 411 is the trailing shank portion and the second shank portion 412 is the leading shank portion.

The shank 410 is effectively identical to the alternate form of the shank 210 of the compression bone screw 200 of the second embodiment apart from having a central third shank portion 470 separating the longitudinally opposing first and second shank portions 411,412. The central third shank portion 470 is here generally cylindrical in form. The first shank portion 411 is externally threaded with a first shank thread 413, whilst the second shank portion 412 is externally threaded with a second shank thread 416. The first and second shank threads 413, 416 are opposite-handed. In the arrangement depicted, as per the alternate form of the compression bone screw 200 of the second embodiment, the first shank thread 413 is a right-handed thread whereas the second shank thread 416 is a left-handed thread. It is envisaged that the first shank thread 413 may be a left-handed thread and the second shank thread 416 may be a right-handed thread, although this would require rotational driving of the shank in opposing directions to those direction discussed below during the first and second modes of operation. In the arrangement depicted, the shank 410 again has an overall diameter of approximately 6 mm and the first and second shank threads 413, 416 have a pitch of approximately 1 mm.

So as to retain the first and second elements 420, 430 on the shank 410, and specifically to lock the first and second elements 420, 430 relative to the shank 410 during the first mode of operation discussed below, first and second primary detents 418,419, in the form of first and second enlarged heads are formed on (or fitted to) the end of each of the first and second shank portions 411, 412. First and second secondary detents 471, 472 are formed at the opposing ends of the third shank portion 470 to limit travel of the first and second members 420, 430, along the first and second shank portions 411, 412 respectively, thereby limiting the amount of compression able to be applied by the compression bone screw 400. The first and second secondary detents 471, 472 are formed by the runout of the first and second shank threads 413, 416 at their junction with the third shank portion 470 which here has a diameter approximately equal to the overall diameter of the first and second shank portions 411, 412. A bore hole 441 extends through the longitudinal length of the shank 410 for receipt of a guide wire during implantation.

The first element 420 has a first element aperture 421 longitudinally extending therethrough and a first element internal thread 422 formed on the wall of the first element aperture 421. The first element internal thread 422 matches the first shank thread 413, enabling the first element 420 to be threadingly mounted on the first shank portion 411. The first element 420 is externally threaded with a first element external thread 423 which is like-handed to the first shank thread 413, here being a right-handed thread. If the first shank thread 413 were configured as a left-handed thread, the first element external thread 423 would generally be a left-handed thread. The first element external thread 423 extends along substantially the entire length of the first element 420 and is again a self-tapping thread having a relatively coarse pitch of approximately 2 mm and depth of approximately 1.5 mm. A first element recess 428 is formed in the trailing end of the first element 420 for receipt of the first primary detent 418. The base of the first element recess 428 forms an annular first element shoulder 429 that engages the first primary detent 418 at one end of the extent of travel of the first element 420 along the first shank portion 411. Travel of the first element 420 along the first shank portion 411 in the opposing direction is limited by engagement of the leading end of the first element 420 with the first secondary detent 471 defined at the junction between the first shank portion 411 and the third shank portion 470. The first element recess 428 has a length such that the first primary detent 418 does not project beyond the first element recess 428 at any point along the extent of travel of the first element 420, ensuring that there is no overhang of the shank 410 from the first element 420 upon implantation.

The second element 430 is effectively arranged as a mirror image of the first element 420. The second element 430 has a second element aperture 431 longitudinally extending therethrough and a second element internal thread 432 formed in the wall of the second element aperture 431 that matches the second shank thread 416 for mounting the second element 430 on the second shank portion 412. The second element 430 is externally threaded with a second element external thread 433 which is like-handed to the first shank external thread 423, here being a right-handed thread. The second element external thread 433 extends along substantially the entire length of the second element 430 and is again a self-tapping thread having a relatively coarse pitch of approximately 2 mm (here substantially identical to the pitch of the first element external thread 423) and depth of approximately 1.5 mm. A second element recess 438 is formed in the leading end of the second element 430 for receipt of the second primary detent 419. The base of the second element recess 438 forms an annular second element shoulder 439 that engages the second primary detent 419 at one end of the extent of travel of the second element 430 along the second shank portion 412. Travel of the second element 430 along the second shank portion 412 in the opposing direction is limited by engagement of the trailing end of the second element 430 with the second secondary detent 472 defined at the junction between the second shank portion 430 and the third shank portion 470. The second element recess 438 has a length such that the second primary detent 419 does not project beyond the second element recess 438 at any point along the extent of travel of the second element 430.

In the arrangement depicted, the first and second elements 420, 430 again have an overall diameter of approximately 11 mm and a length of the order of 15 mm.

As with the compression bone screw 200 of the second embodiment, the compression bone screw 400 of the fourth embodiment is configured such that the shank 410, first element 420 and second element 430 operate in a first mode of operation in which the shank 410, first element 420 and second element 430 are rotationally driven in unison and in a second mode of operation in which the shank 410 is rotationally driven independent of the first and second elements 420, 430. As with the alternate form of the compression bone screw 200 of the second embodiment, the two modes of operation are provided for by releasably locking the first and second elements 420, 430 to the shank 410 by way of engagement of the first and secondary detents 418, 419 at the opposing ends of the shank 410 with the first and second element shoulders 429, 439 defined in the first and second element recesses 428, 438 during the first mode of operation, as will be further discussed below.

The end face 414 of the first shank portion 411 is provided with a drive formation, depicted in Figure 29 in the form of a drive slot 415 for rotationally driving the shank 410 in both the first and second modes of operation. The drive formation may again take any suitable form, including a hex socket or star drive.

The fixing of proximal and distal bone fragments 1, 2 of a fractured bone using the compression bone screw 400 of the fourth embodiment is generally as per that of the alternate form of the compression bone screw 200 of the second embodiment described above and will now be described with reference to Figures 27 and 28.

The proximal and distal bone fragments 1, 2 are prepared in the same manner as described above by drilling a guide hole through the proximal and distal bone fragments 1, 2. The compression bone screw 400 is then arranged as depicted in Figure 27 with the first and second elements 420, 430 in an extended position with the first and second primary detents 418, 419 engaging the first and second element shoulders 429, 439, thereby locking the first and second elements 420, 430 against further extension relative to the first and second shank portions 411, 412. The compression bone screw 400 is then inserted into the guide hole formed in the proximal bone fragment 1 with the second element 430 leading. A drive tool in the form of a standard screwdriver is engaged with the compression bone screw 400, engaging the drive slot 415 formed in the first shank end face 414. The drive tool is then rotationally driven by hand in a clockwise direction in the first mode of operation. The resulting clockwise rotation of the first shank 410 would normally tend to extend the first and second elements 410, 420 along the first and second shank portions 411, 412, however engagement of the first and second primary detents 418, 419 with the first and second element shoulders 429, 439 prevents longitudinal displacement of the first and second elements 420, 430 in an extension direction along the first and shank portions 411, 412. Accordingly, relative rotation of the first and second elements 420, 430, relative to the shank 410 is prevented and the first and second elements 420, 430 and shank 410 accordingly rotate in unison, advancing the entire compression bone screw 400 through the guide hole. The compression bone screw 400 is rotationally driven until the second element 430 is firmly embedded within the distal bone fragment 2 and the first element 420 is firmly embedded within the proximal bone fragment 1.

The drive tool is then rotated in a counter-clockwise direction in the second mode of operation. With the first and second elements 420, 430 firmly embedded within the distal and proximal bone fragments 1, 2 respectively, the counter-clockwise rotation of the shank 410 results in rotation of the shank 410 independently of the first and second elements 420, 430. The right-handed configuration of the first shank thread 413 tends to advance the first element 420 along the shank 410, thereby advancing the proximal bone fragment towards the distal bone fragment 2. At the same time, the left-handed configuration of the second shank thread 416 tends to move the second element 430 back along the shank 410, thereby drawing the distal bone fragment 2 back towards the proximal bone fragment 1. Accordingly, the proximal and distal bone fragments 1, 2 are drawn towards each other, generating the desired compression between the bone fragments 1, 2 without displacement of either of the first and second elements 420, 430. The maximum degree of compression able to be generated is limited by engagement of the first and second elements 420, 430 with the first and second secondary detents 471, 472 respectively, limiting the relative longitudinal displacement of the first and second elements 420, 430 along the first and second shank portions 411, 412. A torque wrench can also be utilised as with the second embodiment to accurately control the degree of compression generated. When fully implanted, the shank 410 does not overhang the first and second elements 420, 430.

A compression bone screw 500 according to a fifth embodiment is depicted in Figures 30 and 31. The compression bone screw 500 is a modified version of the compression bone screw 400 of the fourth embodiment as described above. Those features of the compression bone screw 500 of the fifth embodiment that are identical, or equivalent, to features of the compression bone screw 400 of the fourth embodiment are again provided with like reference numerals increased by 100. The compression bone screw 500 comprises a shank 510, a first element 520 and a second element 530. In the arrangement depicted, the first element 520 is the trailing element, the first shank portion 511 is the trailing shank portion and the second shank portion 512 is the leading shank portion.

The second element 530 is identical to the second element 430 of the compression bone screw 400 of the fourth embodiment. Similarly, the second shank portion 512 of the shank 510 is identical to the second shank portion 412 of the compression bone screw 400 of the fourth embodiment as described above.

The compression bone screw 500 of the fifth embodiment differs from the compression bone screw 400 of the fourth embodiment by virtue of the configuration of the first element 520, the length of the first shank portion 511 and the length of the third shank portion 570. The compression bone screw 500 is particularly configured for use with a locking plate 580 as will be discussed further below.

The first element 520 is shorter than the first element 420 of the compression bone screw 400 of the fourth embodiment, but otherwise has the same general form except that the first element external thread 523 is a thread that matches a first plate thread 581 of a first plate aperture 582 extending through the locking plate 580. The first element external thread 523 will typically have a small taper so that it locks into the first plate aperture 582 by an interference fit. The first element external thread 523 is again a right-handed thread, being like-handed to the second element external thread 533. The first element external thread 523 will, however, typically have a finer pitch than that of the second element external thread 523, to match the pitch of the first plate thread 581. The first element 520 also has a first element aperture 521 longitudinally extending therethrough and a first element internal thread 522 formed on the wall of the first element aperture 521. The first element internal thread 522 matches the first shank thread 513, enabling the first element 520 to be threadingly mounted on the first shank portion 511. A first element recess 528 is formed in the trailing end of the first element 520 for receipt of a first primary detent 518 in the same manner as the first primary detent 418 of the compression bone screw 400 of the fourth embodiment, with the base of the first element recess 528 forming an annular first element shoulder 529 that engages the first primary detent 518 at one end of the extent of travel of the first element 520 along the first shank portion 511.

As will be appreciated from the representations, the non-threaded third shank portion 570 has an increased length compared to that of the third shank portion 470 of the compression bone screw 400 of the fourth embodiment, having a sufficient length to extend through the thickness of the proximal bone fragment 1 in operation.

The first shank portion 511 is of identical configuration to the first shank portion 411 of the compression bone screw 400 of the fourth embodiment, except that it has a shortened length in keeping with the shortened length of the first element 520. The first shank portion 511 is externally threaded with the first shank thread 513 which, in the arrangement depicted, is again a right-handed thread which is opposite-handed to the left-handed second shank thread 516. The first shank portion 511 is again provided with the first primary detent 518 in the form of a first enlarged head formed on the end of the first shank portion 511. A first secondary detent 571 is also formed at the adjoining end of the third shank portion 570.

Use of the compression bone screw 500 with a locking plate 580 would be particularly suitable to augment fixation, especially in the fixation of large bones such as the femur which is generally represented in Figures 30 and 31. Use of a locking plate 580 would also be particularly suitable in the fixation of arthodeses. Use of a locking plate for bone fixation is a known procedure that has the advantage of providing additional angular stability to the fixation. The use of typical known bone screws with locking plates, however, does not readily allow application of the desired compression to the fixation as the bone screw is generally not able to advance any further once the threaded screw head engages the plate thread of the plate aperture extending through the locking plate.

The fixing of proximal and distal bone fragments 1, 2 of a fractured bone using the compression bone screw 500 of the fifth embodiment, together with a locking plate 580, will now be described with further reference to Figures 30 and 31.

Again a guide hole is drilled through the proximal and distal bone fragments 1, 2. A further guide hole is also drilled in a stable bone portion 4 of the bone. A further, typically single part, bone screw 590 is then used to secure the locking plate 580 to the stable bone portion 4, with the bone screw 590 threadingly engaging both the stable bone portion 4 and the locking plate 590, passing through a threaded second plate aperture 583 of the locking plate 580.

The compression bone screw 500 is then arranged as depicted in Figure 30 with the first and second elements 520, 530 in the extended position with the first and second primary detents 518, 519 engaging the first and second element shoulders 529, 539, locking the first and second elements 520, 530 against further extension relative to the first and second shank portions 511, 512. The compression bone screw 500 is inserted through the first plate aperture 582 and the guide hole in the bone fragments 1, 2 with the second element 530 leading. The drive tool, again in the form of a standard screwdriver, is engaged with the compression bone screw 500, engaging a drive slot formed in the first shank end face 514. The drive tool is rotationally driven by hand in a clockwise direction in the first mode of operation. Engagement of the first and second primary detents 518, 519 with the first and second element shoulders 529, 539 again prevents longitudinal displacement of the first and second elements 520, 530 along the first and second shank portions 511, 512 during this first mode of operation. The first and second elements 520, 530 and shank 510 accordingly rotate in unison, advancing the entire compression bone screw 500 through the first plate aperture 582 and the guide hole in the bone fragments 1, 2. The compression bone screw 500 is rotationally driven until the second element 530 is firmly embedded within the distal bone fragment 2 and the first element 520 is embedded within the first plate aperture 582. As noted above, the first element thread 523 will typically be tapered such that the first element 520 is frictionally locked within the first plate aperture 582 when the compression bone screw 500 is driven at a high torque to firmly embed the first element 520.

The drive tool is then rotated in a counter-clockwise direction in the second mode of operation. The counter-clockwise rotation of the shank 510 results in rotation of the shank 510 independently of the first and second elements 520, 530. The right-handed configuration of the first shank thread 513 tends to advance the first element 520 along the shank 510, thereby advancing the locking plate 580 and, as a result, the proximal bone fragment 1 towards the distal bone fragment 2. At the same time, the left-handed configuration of the second shank thread 516 tends to move the second element 530 back along the shank 510, thereby drawing the distal bone fragment 2 back towards the proximal bone fragment 1 and locking plate 580.

Accordingly, the proximal and distal bone fragments 1, 2 are again drawn towards each other, generating the desired compression between the bone fragments 1, 2 without displacement of the first element 520 within the locking plate 580 or the second element 430 within the distal bone fragment 2. The maximum degree of compression able to be generated is again limited by engagement of the first and second elements 520, 530 with the first and second secondary detents 571, 572 in a similar manner to that described above in relation to use of the compression bone screw 400 of the fourth embodiment.

Each of the components of each of the compression bone screws described above may be formed of stainless steel, titanium or any other suitable biologically inert engineering material, including bioabsorbable materials such as polylactic acid or the like. The various dimensions of each of the components of each of the compression bone screws described above may also be adjusted to suit a particular intended application.

A person of ordinary skill in the art will appreciate that various other modification and alterations of the compression screws described may be made. In particular, various other mechanisms may be utilised to provide for rotation of the shank with the first (and second) elements in the first mode of operation.

## Claims

1. A compression bone screw (100) comprising: a shank (110) having longitudinally opposing first and second shank portions (111,112), said first shank portion being externally threaded with a first shank thread (113); a first element (120) threadingly mounted on said first shank portion (111) by way of a first element internal thread (122) threadingly engaging said first shank thread (113), said first element being externally threaded with a first element external thread (123); a second element (130) mounted on, or integrally formed with, said second shank portion (112), said second element (130) being externally threaded with a second element external thread (133), said second element external thread (133) and said first element external thread (123) being like-handed; and said first shank portion and said first element being configured to operate in a first mode of operation in which said shank and said first element are rotationally driven in unison and a second mode of operation in which said shank is rotationally driven independent of said first element, such that said first element moves along said first shank portion towards said second element; wherein a pitch of said first element external thread (123) is substantially equal to a pitch of said second element external thread (133); **characterised in that** the said first element external thread (123) is opposite-handed to said first shank thread (113).

2. The screw of claim 1, wherein said first element external thread and said second element external thread are each self-tapping threads.

3. The screw of claim 1 , wherein said pitch of said first and second element external threads is coarser than a pitch of said first shank thread.

4. The screw of claim 1 , wherein said first shank portion is a trailing shank portion, said second shank portion is a leading shank portion, said first element is a trailing element and said second element is a leading element.

5. The screw of claim 4, wherein said second element is fixedly mounted on, or integrally formed with, said second shank portion.

6. The screw of claim 5, wherein an end face of said first shank portion is provided with a primary drive formation and an end face of said first element is provided with a secondary drive formation, said primary and secondary drive formations being engageable with a drive tool in said first mode of operation for rotationally driving said shank and said first element in unison, said primary drive formation being engageable with the drive tool in said second mode of operation for rotationally driving said shank independently of said first element.

7. The screw of claim 4, wherein said first element external thread has a first element external thread outer diameter that tapers towards a leading end of said first element.

8. The screw of claim 4, wherein at least a leading region of said first shank thread tapers towards a trailing end of said shank and at least a leading region of said first element is configured to radially expand upon engagement with said leading region of said first shank thread.

9. The screw of claim 8, wherein at least a leading region of said first element is provided with one or more longitudinally extending slits to enable radial expansion of said leading region of said first element.

10. The screw of claim 5, wherein said screw further comprises a locking mechanism configured to lock said first element relative to said first shank portion upon installation of said compression bone screw.

11. The screw of claim 10, wherein said locking mechanism comprises a deformable detent secured to one of said first element and said first shank portion such that, upon installation of said compression bone screw, the other of said first element and said first shank portion engages and deforms said deformable detent to lock said first element to said first shank portion.

12. The screw of claim 10, wherein said locking mechanism comprises a locking device configured to engage said end face of said first shank portion and said end face of said first element to lock said first element relative to said first shank portion upon installation of said compression bone screw.

13. The screw of claim 5, wherein said first shank portion and said first element are each configured to releasably engage a locking member to fix said first shank portion relative to said first element for said first mode of operation.

14. The screw of claim 13, wherein said first shank portion is provided with a longitudinally extending shank groove and said first element is provided with a first element groove, said shank grove and said first element groove co-operating to receive said locking member in use.

15. The screw of claim 1, wherein: said second shank portion is externally threaded with a second shank thread, said second shank thread being opposite-handed to said first shank thread, said second element being threadingly mounted on said second shank portion by way of a second element internal thread threadingly engaging said second shank thread; and said second shank portion and said second element are configured to be rotationally driven in unison in said first mode of operation and such that said shank is rotationally driven independent of said second element in said second mode of operation such that said second element moves along said second shank portion towards said first element.

## Patentansprüche

1. Kompressionsknochenschraube (100) mit: einem Schaft (110) mit in Längsrichtung einander gegenüberliegenden ersten und zweiten Schaftabschnitten (111, 112), wobei der erste Schaftabschnitt außen mit einem ersten Schaftgewinde (113) versehen ist; einem ersten Element (120), das über ein Gewinde am ersten Schaftabschnitt (111) mittels eines Innengewindes (122) des ersten Elements befestigt ist, das in das erste Schaftgewinde (113) mit einem Gewinde eingreift, wobei das erste Element außen mit einem Außengewinde (123) des ersten Elements versehen ist; einem zweiten Element (130), das an dem zweiten Schaftabschnitt (112) befestigt oder integral damit gebildet ist, wobei das zweite Element (130) außen mit einem Außengewinde (133) des zweiten Elements versehen ist, wobei das Außengewinde (133) des zweiten Elements und das Außengewinde (123) des ersten Elements gleichläufig ausgebildet sind; und wobei der erste Schaftabschnitt und das erste Element zum Betreiben in einem ersten Betriebsmodus, in dem der Schaft und das erste Element drehend in Übereinstimmung angetrieben werden, und in einem zweiten Betriebsmodus ausgestaltet sind, in dem der Schaft drehend unabhängig von dem ersten Element angetrieben ist, sodass das erste Element sich entlang des ersten Schaftabschnitts hin zum zweiten Element bewegt; wobei eine Steigung des Außengewindes (123) des ersten Elements im Wesentlichen gleich einer Steigung des Außengewindes (133) des zweiten Elements ist; **dadurch gekennzeichnet, dass** das Außengewinde (123) des ersten Elements gegenläufig zu dem ersten Schaftgewinde (113) ist.

2. Schraube nach Anspruch 1, wobei das Außengewinde des ersten Elements und das Außengewinde des zweiten Elements jeweils selbstschneidende Gewinde sind.

3. Schraube nach Anspruch 1, wobei die Steigung der Außengewinde des ersten und zweiten Elements gröber als eine Steigung des ersten Schaftgewindes ist.

4. Schraube nach Anspruch 1, wobei der erste Schaftabschnitt ein nachfolgender Schaftabschnitt ist, der zweite Schaftabschnitt ein führender Schaftabschnitt ist, das erste Element ein nachfolgendes Element ist und das zweite Element ein führendes Element ist.

5. Schraube nach Anspruch 4, wobei das zweite Element fest an dem zweiten Schaftabschnitt befestigt oder integral damit geformt ist.

6. Schraube nach Anspruch 5, wobei eine Endseite des ersten Schaftabschnitts mit einer primären Antriebsformation versehen ist und eine Endseite des ersten Elements mit einer sekundären Antriebsformation versehen ist, wobei die ersten und zweiten Antriebsformationen mit einem Antriebswerkzeug im ersten Betriebsmodus zum drehenden Antreiben des Schafts und des ersten Elements in Übereinstimmung betätigbar sind, wobei die primäre Antriebsformation mit dem Antriebswerkzeug im zweiten Betriebsmodus zum drehenden Antreiben des Schafts unabhängig vom ersten Element betätigbar ist.

7. Schraube nach Anspruch 4, wobei das Außengewinde des ersten Elements einen Außendurchmesser des Außengewindes des ersten Elements hat, der sich zu einem führenden Ende des ersten Elements verjüngt.

8. Schraube nach Anspruch 4, wobei wenigstens ein führender Bereich des ersten Schaftgewindes sich zu einem nachfolgenden Ende des Schafts verjüngt und wenigstens ein führender Bereich des ersten Elements ausgestaltet ist, um sich bei Betätigung mit dem führenden Bereich des ersten Schaftgewindes radial auszudehnen.

9. Schraube nach Anspruch 8, wobei wenigstens ein führender Bereich des ersten Elements mit ein oder mehreren sich in Längsrichtung erstreckenden Schlitzen versehen ist, um eine radiale Expansion des führenden Bereichs des ersten Elements zu ermöglichen.

10. Schraube nach Anspruch 5, wobei die Schraube ferner einen Verriegelungsmechanismus aufweist, der ausgestaltet ist, um das erste Element relativ zum ersten Schaftabschnitt nach Einbau der Kompressionsknochenschraube zu verriegeln.

11. Schraube nach Anspruch 10, wobei der Verriegelungsmechanismus eine verformbare Rastung aufweist, die an einem des ersten Elements und des ersten Schaftabschnitts so befestigt ist, dass nach Einbau der Kompressionsknochenschraube das andere des ersten Elements und des ersten Schaftabschnitts die verformbare Rastung betätigt und deformiert, um das erste Element mit dem ersten Schaftabschnitt zu verriegeln.

12. Schraube nach Anspruch 10, wobei der Verriegelungsmechanismus eine Verriegelungseinrichtung aufweist, die ausgestaltet ist, um die Endseite des ersten Schaftabschnitts und die Endseite des ersten Elements zu betätigen, um das erste Element relativ zum ersten Schaftabschnitt nach Einbau der Kompressionsknochenschraube zu verriegeln.

13. Schraube nach Anspruch 5, wobei der erste Schaftabschnitt und das erste Element jeweils ausgestaltet sind, um lösbar ein Verriegelungselement zu betätigen, um den ersten Schaftabschnitt relativ zum ersten Element für den ersten Betriebsmodus zu fixieren.

14. Schraube nach Anspruch 13, wobei der erste Schaftabschnitt mit einer sich in Längsrichtung erstreckenden Schaftnut versehen ist und das erste Element mit einer Nut des ersten Elements versehen ist, wobei die Schaftnut und die Nut des ersten Elements zusammenwirken, um das Verriegelungselement bei Gebrauch aufzunehmen.

15. Schraube nach Anspruch 1, wobei: der zweite Schaftabschnitt außen mit einem zweiten Schaftgewinde versehen ist, wobei das zweite Schaftgewinde gegenläufig zum ersten Schaftgewinde ist, das zweite Element in Gewindeeingriff an dem zweiten Schaftabschnitt mittels eines Innengewindes des zweiten Elements befestigt ist, das im Gewindeeingriff mit dem zweiten Schaftgewinde steht; und der zweite Schaftabschnitt und das zweite Element ausgestaltet sind, um in Übereinstimmung drehend im ersten Betriebsmodus angetrieben zu werden und so dass der Schaft unabhängig vom zweiten Element im zweiten Betriebsmodus drehend angetrieben ist, sodass das zweite Element sich entlang des zweiten Schaftabschnitts auf das erste Element zu bewegt.

## Revendications

1. Vis (100) de compression pour os comprenant : un corps ayant une première et une seconde parties (111,112) opposées longitudinalement, ladite première partie étant filetée extérieurement avec un premier filetage (113) de corps; un premier élément (120) monté vissant sur ladite première partie de corps au moyen d'un premier filetage (122) coopérant par vissage avec ledit premier filetage (113) de corps, ledit premier élément étant fileté extérieurement avec un filetage (123) externe du premier élément; un second élément (130) monté sur ou venu de fabrication avec ladite seconde partie (112) de corps, ledit second élément (130) étant fileté extérieurement avec un filetage (133) externe du second élément, ledit filetage (133) externe du second élément et ledit filetage (123) externe du premier élément étant de même sens; et ladite première partie de corps et ledit premier élément étant configurés pour fonctionner dans un premier mode de fonctionnement dans lequel ledit premier corps et ledit premier élément sont manoeuvrés de façon synchrone et un second mode de fonctionnement dans lequel ledit premier corps est manoeuvré à rotation de façon indépendante du premier élément, de telle façon que le premier élément se déplace le long du premier élément vers le second élément; dans lequel le pas du filetage (123) externe du premier élément est sensiblement identique au pas du filetage (133) du second élément, **caractérisé en ce que** ledit filetage (123) externe du premier élément est d'un sens opposé à celui du premier filetage (113) de corps.

2. Vis selon la revendication 1, dans laquelle ledit filetage externe du premier élément et ledit filetage externe du second élément ont des filetages auto taraudants.

3. Vis selon la revendication 1, dans laquelle ledit pas desdits filetages externes des premier et second éléments est plus gros que ledit premier filetage de corps.

4. Vis selon la revendication 1, dans laquelle ladite première partie de corps est une partie de corps tractée, ladite seconde partie de corps est une partie de corps de traction, ledit premier élément est un élément tracté et ledit second élément est un élément de traction.

5. Vis selon la revendication 4, dans laquelle ledit second élément est monté de façon solidaire sur ou venu de fabrication avec ladite seconde partie de corps.

6. Vis selon la revendication 5, dans laquelle une extrémité de ladite première partie de corps est munie d'une empreinte de manoeuvre primaire et une face d'extrémité du premier élément est munie d'une empreinte de manoeuvre secondaire, lesdites empreintes primaire et secondaire étant aptes à coopérer avec un outil de manoeuvre dans un premier mode de manoeuvre pour entraîner en rotation lesdits premier et second éléments de corps de façon synchrone, ladite empreinte de manoeuvre primaire étant apte à coopérer avec un outil de manoeuvre dans un second mode de fonctionnement pour manoeuvrer en rotation ledit corps de façon indépendante dudit premier élément.

7. Vis selon la revendication 4, dans laquelle le filetage externe du premier élément a un diamètre extérieur du filetage externe du premier élément qui diminue vers l'extrémité de traction du premier élément.

8. Vis selon la revendication 4, dans laquelle au moins une zone de traction dudit premier filetage de corps diminue vers l'extrémité de traction dudit corps et au moins une zone de traction dudit premier élément est configurée pour s'expanser radialement sous l'engagement de la zone de traction dudit premier filetage de corps.

9. Vis selon la revendication 8, dans laquelle au moins un zone de traction du premier élément est munie d'une ou plusieurs fentes longitudinales d'expansion pour permettre une expansion radiale de la zone de traction du premier élément.

10. Vis selon la revendication 5, dans laquelle ladite vis comprend de plus un mécanisme de verrouillage configuré pour verrouiller ledit premier élément par rapport à la première partie de corps après mise en place de la vis de compression pour os.

11. Vis selon la revendication 10, dans laquelle le mécanisme de verrouillage comprend une butée déformable solidaire de l'un des premier élément et première partie de corps de telle sorte que, une fois la vis de compression pour os mise en place, l'autre des premier élément et première partie de corps coopère et déforme ladite butée déformable pour verrouiller ledit premier élément à ladite première partie de corps.

12. Vis selon la revendication 10, dans laquelle ledit mécanisme de verrouillage comprend un organe de blocage configuré pour coopérer avec ladite première face d'extrémité de la première partie de corps et ladite face d'extrémité dudit premier élément pour verrouiller ledit premier élément par rapport à ladite première partie de corps après mise en place de ladite vis de compression pour os.

13. Vis selon la revendication 5, dans laquelle la première partie de corps et le premier élément sont chacun configurés pour coopérer de façon amovible avec une membre de verrouillage pour solidariser ladite première partie de corps par rapport audit premier élément dans le cas dudit premier mode de fonctionnement.

14. Vis selon la revendication 13, dans laquelle la première partie de corps est munie d'une gorge de corps s'étendant longitudinalement et le premier élément est muni d'une gorge de premier élément, ladite gorge de corps et ladite gorge de premier élément coopérant pour recevoir le membre de verrouillage lors de l'usage.

15. Vis selon la revendication 1, dans laquelle : la seconde partie de corps est filetée extérieurement avec un second filetage de corps, ledit second filetage de corps étant de sens opposé audit filetage du premier filetage de corps, ledit second élément étant monté par vissage sur la seconde partie de corps au moyen d'une filetage interne du second élément coopérant avec le second filetage de corps; et ladite seconde partie de corps et ledit second élément sont configurés pour être manoeuvrés en rotation de façon synchrone dans un premier mode de fonctionnement et pour que ledit corps soit manoeuvré en rotation de façon indépendante du second élément dans un second mode de fonctionnement de façon que le second élément se déplace le long de ladite seconde partie de corps vers ledit premier élément.
